# EUROPEAN PATENT APPLICATION

(11) **EP 2 255 763 A1**
(43) Date of publication of application: **01.12.2010**
(21) Application number: 09725466.8
(22) Date of filing: 19.01.2009
(51) Int. Cl.: A61F 13/15, A61F 13/49, A61F 13/496, A61F 13/511

(54) **DISPOSABLE PANTS-TYPE DIAPER**

(30) Priority: 26.03.2008 JP 2008081759
(71) Applicant: Unicharm Corporation, Ehime 799-0111 (JP)
(72) Inventor: OTSUBO, Toshifumi, Kanonji-shi Kagawa 769-1602 (JP)
(74) Representative: Knights, Rupert
(86) International application number: PCT/JP2009/050617
(87) International publication number: WO 2009/119138

(57) **Abstract**

The present invention aims to provide a disposable pants-type diaper improved so that the separator being well known per se may fulfill its primary function.

A disposable pants-type diaper (1) includes an innermost sheet (20) lying between a liquid-pervious inner sheet (2) covering a bodily fluid-absorbent core and diaper wearer's skin and attached to the inner sheet (2) to form a body waste retaining space (31). The innermost sheet (20) is formed with a front opening (33) allowing the passage of urine into the body waste retaining space (31) and a rear opening (34) allowing the passage of feces into the body waste retaining space (31). A portion of the innermost sheet (20) extending between the front opening (33) and the rear opening (34) droops down and a lower end (35) thereof to form a separator (20a). The separator is provided with a sheet member (81) having a thickness (N) in a range of 1 to 10mm, a width in a range of 10 to 70mm and a height (L) in a range of 30 to 100% of a dimension (K) of the separator (20a) in the vertical direction and functioning to restrict a degree of deformation potentially occurring in the separator.

## Description

### TECHNICAL FIELD

The present invention relates to disposable pants-type diapers with which the wearer's skin would not be soiled with urine and/or feces.

### RELATED ART

It is well known to provide diapers each with a structure serving to prevent urine and feces from being mixed with each other inside the diaper put on the wearer's body. For example, in the case of the disposable pants-type diaper described in JP 2007-296314 A (PATENT DOCUMENT 1), a nonwoven fabric piece defining a separator serving to prevent urine and feces from being mixed with each other is provided in the crotch region and this nonwoven fabric piece is fixed along the opposite side edges thereof to the inner surface of the diaper. The nonwoven fabric piece has a front end and a rear end both extending in the transverse direction of the diaper and these two ends are partially integrated together on a center line bisecting a width dimension of the diaper. The diaper is formed with a body waste retaining space symmetrically in a front-back direction about the spacer which functions to divide this space into front and rear body waste retaining sub-spaces so that urine and feces may be separately retained.
PATENT DOCUMENT 1: JP 2007-296314 A

### DISCLOSURE OF THE INVENTION

### PROBLEM TO BE SOLVED BY THE INVENTION

In the case of this diaper of prior art, the separator may collapse onto the liquid-pervious inner sheet covering the absorbent core as the wearer takes a sitting posture and, in consequence, feces once having been retained in the body waste retaining sub-space defined behind the separator may be partially extruded forward beyond the separator until the wearer's external genital may be soiled with feces. In addition, the core lying in the crotch region may be crooked in the transverse direction and pressed against the wearer's thighs as the wearer moves his or her legs to be closed up to the wearer's skin. Eventually, the internal volume of the body waste retaining space may be unacceptably reduced and the wearer's skin may be soiled with excess feces.

In view of the problem as has been described above, it is a principal object of the present invention to improve the disposable pants-type diaper so that the separator may fulfill its function without being affected by variation of the diaper wearer's posture.

### MEASURE TO SOLVE THE PROBLEM

The object set forth above is achieved, according to the present invention, by an improvement in a disposable pants-type diaper having a front-back direction, a transverse direction and a vertical direction being orthogonal one to another and comprising a crotch region, a front waist region extending forward from the crotch region and a rear waist region extending rearward from the crotch region wherein the crotch region includes a bodily fluid absorbent structure comprising a liquid-pervious inner sheet, a liquid-impervious outer sheet and a bodily fluid absorbent core sandwiched between the inner and outer sheets and curved with inner sheet inside and the crotch region is additionally formed with a separator adapted to separate urine and feces from each other.

The improvement according to the present invention is characterized in aspects as follow. The innermost sheet member bonded to the inner sheet to be interposed between skin of wearer of the diaper and the inner sheet is adapted to be partially spaced upward from the inner sheet in the vertical direction and thereby to form a body waste retaining space having a front opening allowing urine to flow into the body waste retaining space and a rear opening allowing feces to move into the body waste retaining space. A portion of the innermost sheet member extending in the front-back direction between the front opening and the rear opening is folded to be opposed to each other in the front-back direction and directly or indirectly bonded to itself along a center line extending in the transverse direction and bisecting a dimension of the diaper in the front-back direction so that the portion droops down toward the crotch region and a lower end thereof is joined to the inner sheet to form the separator. The separator is provided along the center line with a sheet member having a thickness in the front-back direction in a range of 1 to 10mm, a width in the transverse direction in a range of 10 to 70mm and a height in the vertical direction in a range of 30 to 100% of a dimension of the separator in the vertical direction and functioning to restrict a degree of deformation potentially occurring in the separator formed of the innermost sheet material.

According to one preferred embodiment of the present invention, the sheet member has a level of stiffness in a range of 40 to 130N based on Procedure D of Stiffness Measuring Method specified by Section 6 of JIS K 6400-2.

According to another preferred embodiment of the present invention, the sheet member is attached to the separator on a front surface of the separator, a rear surface of the separator or between the opposed and joined portions of the innermost sheet member.

### EFFECT OF THE INVENTION

In the disposable pants-type diaper according to the present invention, the innermost sheet member forming the separator adapted to separate urine and feces from each other and thereby to prevent urine and feces from being mixed with each other is provided with the sheet member having a thickness in the front-back direction in a range of 1 to 10mm, a width in the transverse direction in a range of 10 to 70mm and a height in the vertical direction in a range of 30 to 100% of a dimension of the separator in the vertical direction and functioning to restrict the separator from being deformed. With this arrangement, the separator can fulfill its primary function without being readily crooked even if the diaper wearer's posture changes in various ways. The separator provided with this sheet member serves also to restrict the bodily fluid-absorbent core from being crooked to be pressed against the diaper wearer's skin. In this way, the separator is able to prevent the internal volume of the body waste retaining space from being unacceptably reduced due to such crooking of the core.

### BRIEF DESCRIPTION OF THE DRAWINGS

[FIG. 1] Fig. 1 is a partially cutaway perspective view of a pants-type diaper according to the present invention.
[FIG. 2] Fig. 2 is a plan view showing the pants-type diaper having front and rear waist regions thereof unsealed from each other and flatly developed.
[FIG. 3] Fig. 3 is a sectional view taken along the line III-III in Fig. 1.
[FIG. 4] Fig. 4 is an overhead view of the pants-type diaper as viewed from above in a direction indicated by the arrows IV-IV in Fig. 3.
[FIG. 5] Fig. 5 is a partially cutout perspective view of one preferred embodiment of the pants-type diaper according to the present invention.
[FIG. 6] Fig. 6 is a sectional view taken along the line VI-VI in Fig. 5.
[FIG. 7] Fig. 7 is a view similar to Fig. 6, showing another preferred embodiment of the present invention.

### IDENTIFICATION OF REFERENCE NUMERALS USED IN THE DRAWINGS

- 1: pants-type diaper
- 2: inner sheet
- 3: outer sheet
- 4: absorbent core
- 6: crotch region
- 6a: lowermost portion of crotch region
- 7: front waist region
- 8: rear waist region
- 10: chassis
- 12: leg-openings
- 20: innermost sheet member
- 20a: separator
- 31: body waste retaining space
- 33: front opening
- 34: rear opening
- 35: lower end
- 45: liquid-absorbent structure
- 81: sheet member (deformation restricting sheet)
- 120: innermost sheet member
- A: front-back direction
- B: transverse direction
- C: vertical direction
- K: dimension of separator in vertical direction
- L: height
- M: width
- N: thickness
- P-P: center line

### DETAILED DESCRIPTION OF THE PREFERRED EMBODIMENTS

Details of the disposable pants-type diaper according to the present invention will be more fully understood from the description given hereunder with reference to the accompanying drawings.

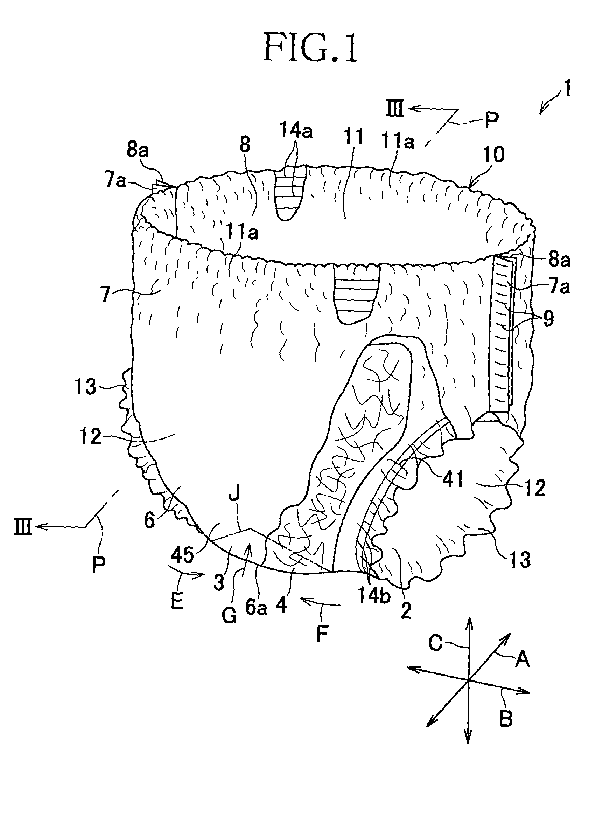

Fig. 1 is a partially cutaway perspective view showing the disposable pants-type diaper 1 as put on the wearer's body wherein a front-back direction, a transverse direction and a vertical direction of the diaper 1 are respectively indicated by the double-headed arrows A, B and C which are orthogonal one to another. The diaper 1 includes a pants-shaped chassis 10 comprising a liquid-pervious inner sheet 2, a liquid-impervious outer sheet 3 and a bodily fluid-absorbent core 4. The chassis 10 has a crotch region 6, a front waist region 7 extending forward from the crotch region 6 and a rear waist region 8 extending rearward from the crotch region 6. The front and rear waist regions 7, 8 are put flat and sealed together along respective pairs of side edges 7a, 7a and 8a, 8a of these waist regions 7, 8, more specifically, at joints 9 arranged intermittently in the vertical direction as viewed in Fig. 1 along these side edges 7a, 7a and 8a, 8a and thereupon a waist-opening 11 and a pair of leg-openings 12 are formed. Along a periphery 11a of the waist-opening 11, a plurality of waist elastic members 14a extending in a circumferential direction is sandwiched between the inner and outer sheets 2, 3 and bonded under tension to at least one of the inner and outer sheets 2, 3. Along a periphery of each of the leg-openings 12, a plurality of leg elastic members 14b extending in the circumferential direction is sandwiched between the inner and outer sheets 2, 3 and bonded under tension to at least one of these inner and outer sheets 2, 3. Three components, i.e., the leg elastic members 14b and the inner and outer sheets 2, 3 cooperate together to define respective elastic regions 41 surrounding the wearer's legs. Of the diaper 1, at least the crotch region 6 includes a bodily fluid absorbent structure 45 comprising the inner and outer sheets 2, 3 and the absorbent core 4 sandwiched therebetween. The bodily fluid absorbent structure 45 extends in the front-back direction A and curves convexly downward to define a bottom portion 6a of the crotch region 6.

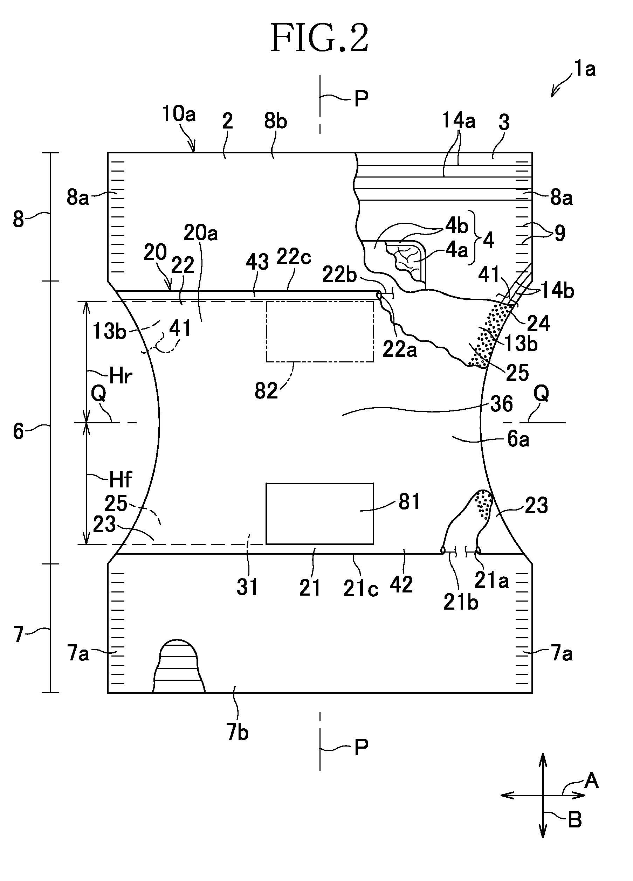

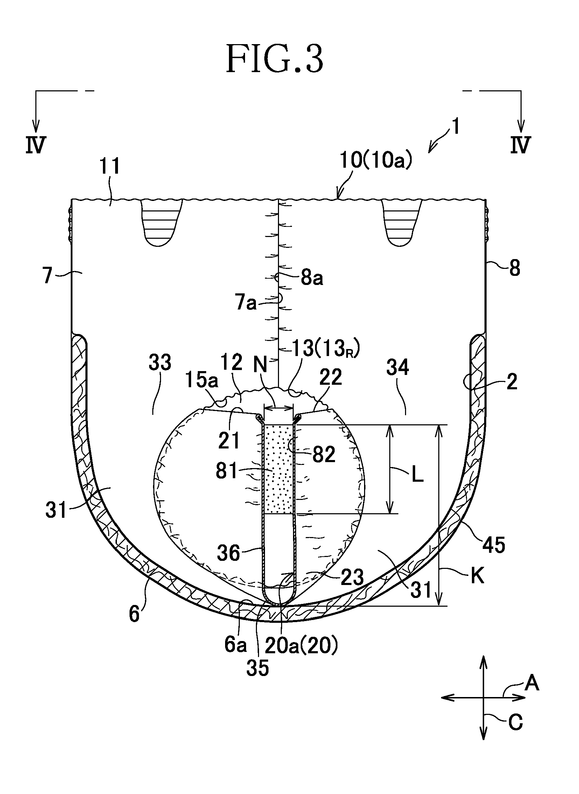

Fig. 2 is a partially cutaway perspective view showing the diaper 1a as after the front and rear waist regions 7, 8 of the diaper 1 have been unsealed at the joints 9 and flatly developed as a whole in the front-back direction A as well as in the transverse direction B wherein such developed diaper 1a is shown as viewed from its inner side. In the state of such developed diaper 1a, the chassis 10 in Fig. 1 appears as a concave shaped flattened chassis 10a. The periphery 11a of the waist-opening 11 in Fig. 1 appears herein as a front end 7b and a rear end 8b of the flattened chassis 10a and the peripheries 13 of the respective leg-openings 12 appear herein as the crotch region's side edges 13b of the flattened chassis 10a. While the crotch region's side edges 13b convexly curve toward the center line P-P extending in the front-back direction A to bisect a dimension of the flattened chassis 10a in the transverse direction X, the respective pairs of side edges 7a, 7a and 8a, 8a of the front and rear waist regions 7, 8 extend substantially in parallel to this center line P-P. The absorbent core 4 also is concave shaped and is formed of a mixture 4a of fluff pulp fibers and super-absorbent polymer particles wrapped with a bodily fluid-absorbent and highly dispersant wrapping sheet 4b such as a tissue paper. In the flattened chassis 10a, the innermost sheet member 20 made of, for example, a hydrophobic nonwoven fabric, a plastic film or a composite sheet thereof, more preferably, a hydrophobic and liquid-impervious nonwoven fabric, a plastic film or a composite sheet thereof is attached to the inner sheet 2 defining the inner surface of the flattened chassis 10a in the crotch region 6 so that the innermost sheet member 20 may define a separator 20a (See Fig. 3). Such flatly developed diaper 1a is formed symmetrically about the center line P-P extending in the front-back direction A. The lowermost portion 6a of the crotch region 6 lies on the transverse center line Q-Q bisecting a dimension of the flatly developed diaper 1a in the front-back direction A.

The innermost sheet member 20 is attached to the flattened chassis 10a so that the innermost sheet member 20 may be interposed between the inner sheet 2 and the diaper wearer's skin (not shown) when the diaper 1 is put on the wearer's body. The innermost sheet member 20 has side edges 23 respectively fixed to the crotch region's side edges 13b by hot melt adhesive 24, a front edge 21 lying before the lowermost portion 6a of the crotch region 6 and extending in the transverse direction B between the side edges 23, 23 and a rear edge 22 lying behind the lowermost portion 6a of the crotch region 6 and extending in the transverse direction B between the side edges 23, 23. The innermost sheet member 20 in the illustrated embodiment is not bonded to the inner sheet 2 and adapted to be spaced from the inner sheet 2 except the side edges 23 so that a tunnel- or pocket-like body waste retaining space 31 extending from the front edge 21 to the rear edge 22 may be defined between the innermost sheet member 20 and the inner sheet 2. Along the front edge 21 and the rear edge 22, the innermost sheet member 20 is folded to form a sleeve 21a and a sleeve 22a, respectively. These sleeves 21a, 22a contain therein a front elastic member 21b and a rear elastic member 22a attached under extension thereto, respectively, to define front elastic region 42 and a rear elastic region 43 both extending between the crotch region's side edges 13b. These elastic regions 42, 43 intersect with the elastic regions 41 of the flattened chassis 10a. A distance Hf from the transverse center line Q-Q to the front edge 21 is substantially equal to a distance Hr from the transverse center line to the rear edge 22.

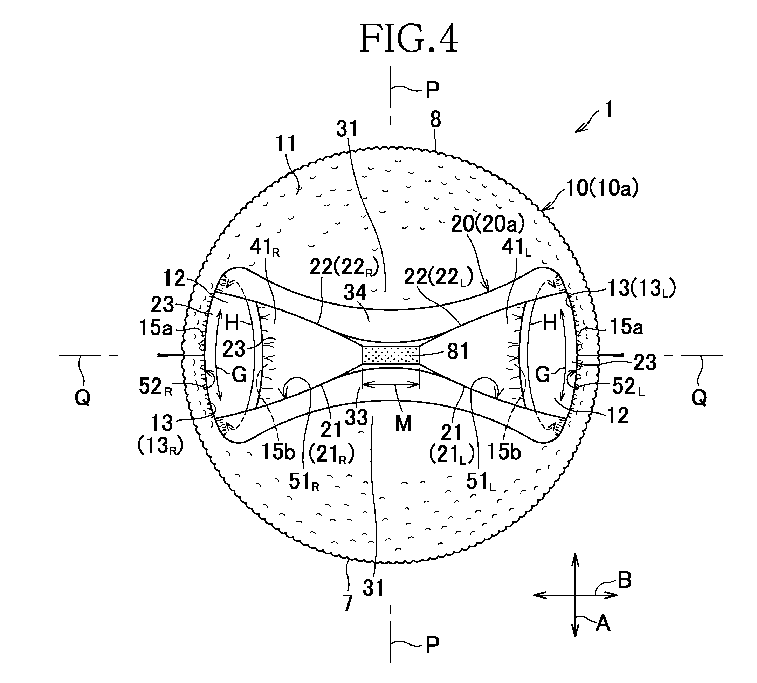

The innermost sheet member 20 is provided on the front edge 21 with a deformation restricting sheet member 81 on the center line P-P extending in the front-back direction A. The deformation restricting sheet member 81 may be provided in the form of a block made of a sponge, a foamed polyurethane, a foamed polystyrene, a foamed polyethylene, a felt or a nonwoven fabric having a flexibility sufficient to be easily deformed under a body weight of the wearer of the diaper 1 and, more preferably, having an elastic recovery properties also sufficient for a rapid recovery after deformation. The innermost sheet member 20 is provided on the rear edge 22 with a region 82 indicated by a imaginary line. The deformation restricting sheet member 81 is bonded to this region 82 when the side edges 7a, 7a and 8a, 8a of the flattened chassis 1a are joined together to obtain the diaper 1 (See Figs. 3 and 4).

Fig. 3 is a sectional view taken along the line III-III in Fig. 1 and the line III-III corresponds to the center line P-P extending in the front-back direction A in Fig. 2. In the pant-shaped chassis 10, the front and rear waist regions 7, 8 are joined together along the respective side edges 7a, 7a and 8a, 8a thereof. The crotch region 6 curves in U-shape as viewed in the front-back direction A and the lowermost portion 6a of the crotch region 6 defines a bottom of this U-shaped curve. The crotch region's side edges 13b appear in Fig. 3 as the peripheral edges 13 defining the leg-openings 12. The innermost sheet member 20 has the front edge 21 and the rear edge 22 thereof bonded to each other by the deformation restricting sheet member 81 and the portion 36 shown in Fig. 2 to extend in the front-back direction A between the front edge 21 and the rear edge 22 sags toward the lowermost portion 6a to form the separator 20a partitioning the crotch region 6 into a front half and a rear half. The deformation restricting sheet member 81 has a thickness N in the front-back direction A and a height L in the vertical direction and is attached to the innermost sheet member 20 by adhesion or sealing. The region in which the deformation restricting sheet member 81 is joined to the rear edge 22 is designated by a reference numeral 82. The separator 20a has its lower end 35 bonded to the lowermost portion 6a of the crotch region 6 by adhesive (not shown) and has height K corresponding to a dimension from the deformation restricting sheet member 81 to the inner sheet 2 in the lowermost portion 6a of the crotch region 6. In the body waste retaining space 31 formed by cooperation of the innermost sheet member 20 with the inner sheet 2, the front edge 21 of the innermost sheet member 20 is adapted to be spaced from the inner sheet 2 and thereby to define a front opening 33 and the rear edge 22 is adapted to be spaced from the inner sheet 2 and thereby to define a rear opening 34.

Fig. 4 is an overhead view of the diaper 1 of Fig. 1 as viewed from above the waist-opening 11 in a direction indicated by the arrows IV-IV in Fig. 3. The deformation restricting sheet member 81 has a width M in the transverse direction B and each of the front and rear ends 21, 22 is bisected in the transverse direction B by the deformation restricting sheet member 81 divided in two, i.e., right segments 21_{R}, 22_{R} adapted to be put in contact with a right leg of the diaper wearer (not shown) and left segments 21_{L}, 22_{L} adapted to be put in contact with a left leg of the diaper wearer. The right segments 21_{R}, 22_{R} cooperate with each other to define a laterally facing V-shaped space so that the wearer's right leg may be guided through this laterally facing V-shaped space. In a similar manner, the left segments 21_{L}, 22_{L} cooperate with each other to define a laterally facing V-shaped space so that the wearer's left leg may be guided through this laterally facing V-shaped space. As illustrated, the respective leg-openings 12 of the chassis 10 have a right leg peripheral edge 13_{R} and a left leg peripheral edge 13_{L}. The respective peripheral edges 13_{R}, 13_{L} comprise upper segments 15a not overlapping the side edges 23 of the innermost sheet member 20 and lower segments 15b overlapping the side edges 23 and bonded thereto (See Fig. 3 also). In Fig. 4, respective ranges of these upper segments 15a and lower segments 15b are indicated by double-headed arrows G and H.

Sequence in which the diaper 1 may be put on the wearer's body will be described below together with behavior of the diaper 1. First, the front and rear waist regions 7, 8 of the chassis 10 may be spaced from each other in the front-back direction A and then the waist-opening 11 may be sufficiently broadened as seen in Figs. 1 and 4. Thereby the front edge 21 of the separator 20a formed of the innermost sheet member 20 is deformed so that the right segment 21_{R} and the left segment 21_{L} are curved in V-shape facing forward in the front-back direction A around the deformation restricting sheet member 81. In a similar manner, the rear edge 22 is deformed so that the right segment 22_{R} and the left segment 22_{L} are curved in V-shape facing rearward in the front-back direction A (See Fig. 4). Consequently, the front opening 33 and the rear opening 34 for the body waste retaining space 31 are automatically broadened (See Fig. 3). The left segments 21_{L}, 22_{L} are spaced from each other in the front-back direction A at the same moment that the right segments 21_{R}, 22_{R} are spaced from each other in the front-back direction A. Now the diaper wearer (not shown) may guide his or her right leg into an opening 41_{R} for the right leg defined by the upper segment 15a of the right leg peripheral edge 13_{R}, the right segment 21_{R} of the front edge 21 and the right segment 22_{R} of the rear edge 22. Then the wearer may guide his or her right leg into the leg-opening 12 for the right leg defined by the upper segment 15a and the lower segment 15b of the right leg peripheral edge 13_{R}. Without interruption, the wearer may guide his or her left leg into an opening 41_{L} for the left leg defined by the upper segment 15a of the left leg peripheral edge 13_{L}, the left segment 21_{L} of the front edge 21 and the left-hand segment 22_{L} of the rear edge 22. Then the wearer may guide his or her right leg into the leg-opening 12 for the left leg defined by the upper segment 15a and the lower segment 15b of the left leg peripheral edge 13_{L}.

In the diaper 1 after it has been put on the wearer's body, the peripheral edges 13 of the respective leg-openings 12, i.e., the right leg peripheral edge 13_{R} and the left leg peripheral edge 13_{L} are elastically contractible in a circumferential direction thereof, on one hand, and the front edge 21 and the rear edge 22 of the separator 20a are elastically contractible in the transverse direction B, on the other hand. With such arrangement, the upper segment 15a of the peripheral edge 13_{R} for the right leg and the right segments 21_{R}, 22_{R} of the separator 20a are elastically put in close contact around the right leg in the vicinity of the right leg's inguinal region to form a primary seal 51_{R} (See Fig. 4) serving to prevent leakage of bodily fluids. Below the primary seal 51_{R}, the lower segment 15b and the upper segment 15a of the peripheral edge 13_{R} for the right leg are integrated to form a secondary seal 52_{R} (See Fig. 4) serving to prevent leakage of bodily fluids. Function of this secondary seal 52_{R} is similar to that of the leg seal in the conventional pants-type diaper. The arrangement and function as have been described just above are true with respect to the left leg. Specifically, the upper segment 15a of the peripheral edge 13_{L} for the left leg and the left segments 21_{L}, 22_{L} of the separator 20a are elastically put in close contact around the left leg in the vicinity of the left leg's inguinal region to form a primary seal 51_{L} serving to prevent leakage of bodily fluids. Below the primary seal 51_{L}, the lower segment 15b and the upper segment 15a of the peripheral edge 13_{L} for the left leg are integrated to form a secondary seal 52_{L} serving to prevent leakage of bodily fluids. The diaper 1 having been put on the wearer's body may be sufficiently pulled up along the body to assure that a central segment of the separator 20a as viewed in the transverse direction B along which the deformation restricting sheet member 81 is attached thereto comes in contact with the wearer's skin between the wearer's external genital and anus.

With the diaper 1 put on the wearer's body in the state as has been described above, the portion 36 of the separator 20a is positioned to divide the wearer's crotch region into a front half and a rear half. In the front half of the wearer's crotch region, the wearer's external genital lying before the front edge 21 is exposed in the front opening 33 and, in the rear half of the wearer's crotch region, the wearer's anus lying behind the rear edge 22 is exposed in the rear opening 34. Consequentially, it is assure that urine discharged by the wearer flows through the front opening 33 into the pocket-like body waste retaining space 31 and feces excreted by the wearer moves through the rear opening 34 into the body waste retaining space 31. In this way, it is possible for the diaper 1 to prevent urine and/or feces from coming in contact with the wearer's skin by the presence of the separator 20a. In addition, the deformation restricting sheet member 81 attached to the separator 20a functions to restrict the bodily fluid-absorbent structure from being crooked along the center line P-P in the front-back direction and thereby pressed against the diaper wearer' skin as the diaper wearer moves his or her legs to be closed up. Thereby the deformation restricting sheet member 81 prevents the internal volume of the body waste retaining space 31 from being unacceptably reduced. The deformation restricting sheet member 81 further prevents the bodily fluid-absorbent structure 45 from moving close to the front opening 33 and/or the rear opening 34 and thereby closing these openings 33, 34.

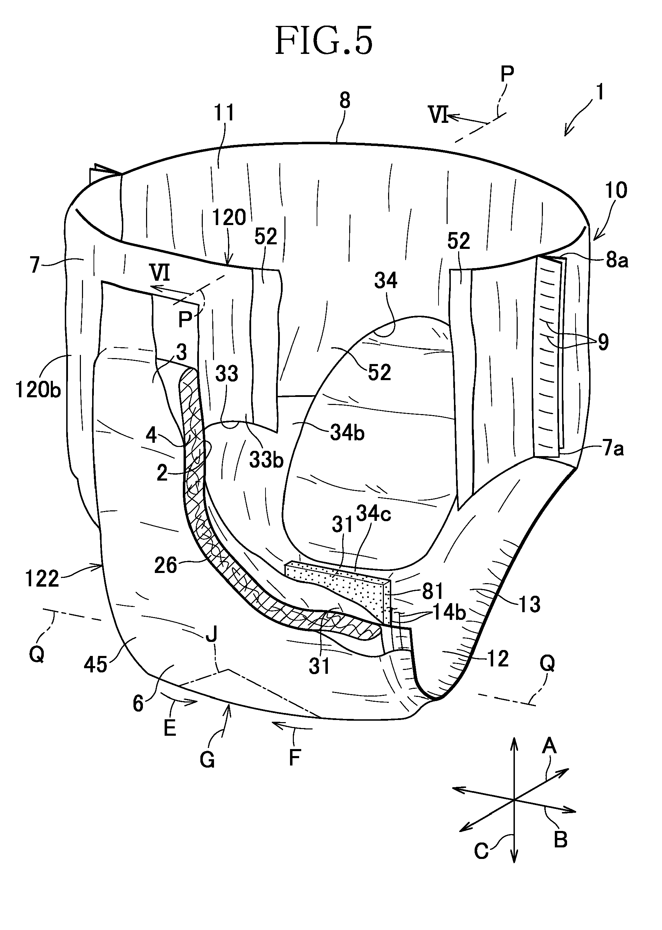

The shape of the bodily fluid-absorbent structure 45 behaving to be crooked in this manner is indicated by imaginary lines in Fig. 1 and Fig. 5. The bodily fluid-absorbent structure 45 in Fig. 1 as well as the bodily fluid-absorbent structure 122 in Fig. 5 corresponding to the bodily fluid-absorbent structure 45 potentially behaves to move in opposite directions of the arrows E and F to be crooked and pressed in the direction of the arrow G against the wearer's skin. However, the deformation restricting sheet member 81 is able to restrict such movement.

In response to change of the diaper wearer's posture from sitting posture to standing posture, the deformation restricting sheet member 81 accelerates the separator 20a to return from its state collapsing on the inner sheet 2 during sitting posture of the diaper wearer to the initial state in which the separator 20a extends downward from the region defined between the front and rear openings 33, 34 toward the inner sheet 2 as shown in Fig. 3 and thereby allows the separator 20a to fulfill its primary function. In the case of the separator 20a extending downward as seen in Fig. 3 having its lower end 35 bonded to the lowermost portion 6a of the crotch region 6, the separator 20a extending between the front edge 21 and the lower end 35, i.e., between the rear edge 22 and the lower end 35 can quickly recover its normal state as seen in Fig. 3 even if the separator 20a has collapsed onto the inner sheet 2. Specifically, the deformation restricting sheet member 81 having stiffness higher than the innermost sheet member 20 is attached to the separator 20a and therefore the separator 20a can easily recover its primary state merely as the front edge 21 is pulled forward and the rear edge 22 is pulled rearward.

The deformation restricting sheet member 81 functioning in the manner as has been described above has the width M preferably in a range of 10 to 70mm, more preferably in a range of 30 to 60mm, the thickness N preferably in a range of 1 to 10mm, more preferably in a range of 3 to 7mm and the height L preferably in a range of 30 to 100%, more preferably in a range of 50 to 80% of the height K of the separator 20a. If any one or more of these dimensions of the deformation restricting sheet member 81 is or are out of the ranges as have been described, feeling to wear the diaper 1 will be deteriorated and/or deformation restricting function of the deformation restricting sheet member 81 in the separator 20a and the bodily fluid-absorbent structure 45 will be insufficient. In order that the deformation restricting sheet member 81 can fulfill its primary function of enhancing the stiffness of the separator 20a in the central segment thereof as viewed in the transverse direction B, the deformation restricting sheet member 81 is formed of a material preferably having stiffness in a range of 40 to 130N, more preferably in a range of 60 to 90N based on Procedure D of Stiffness Measuring Method specified by Section 6 of JIS K 6400-2. If the stiffness is less than 40N, the deformation restricting sheet member 81 will be too flexible to fulfill its function and if the stiffness exceeds 130N, the deformation restricting sheet member 81 will make the separator 20a locally stiff beyond an allowable limit. In addition to the requirements as have been described above, the deformation restricting sheet member 81 preferably has an elastically restoring force.

In the diaper 1, the front edge 21 of the innermost sheet member 20 defining the separator 20a partially defines the peripheral edge of the front opening 33. As viewed in the front-back direction A, the front edge 21 lies behind the wearer's external genital and positioned so that the external genital can be reliably exposed in the front opening 33. While the front edge 21 is exemplarily illustrated to extend linearly in the transverse direction B, it is also possible to form the front edge 21 to curve convexly toward the transverse center line Q-Q. The rear edge 22 of the innermost sheet member 20 partially defines the peripheral edge of the rear opening 34. As viewed in the front-back direction A, the rear edge 22 lies before the wearer's anus and positioned so that the anus can reliably face the rear opening 34. While such rear edge 22 is exemplarily illustrated to extend linearly in the transverse direction B, it is possible to form the rear edge 22 to curve convexly toward the transverse center line Q-Q.

In the crotch region 6 of the flattened chassis 10a shown by Fig. 2, the inner and outer sheets 2, 3 put flat together define side flaps 25 outside the liquid-absorbent core 4. When the crotch region 6 is curved in U-shape under contraction of the elastic regions 41 and thereupon the diaper 1 is put into the state as illustrated by Fig. 1, the side flaps 25 behave to raise themselves along side edges 4c of the core 4 in the vicinity of the transverse center line Q-Q, i.e., in the vicinity of the lowermost portion 6a of the crotch region 6. The innermost sheet member 20 having the side edges 23 bonded to the inner surface of the elastic regions 41 pulls the side flaps 25 inward in the transverse direction of the diaper 1, i.e., toward the center line P-P extending in the front-back direction as the waist-opening 11 is broadened. In consequence, the behavior of the side flaps 25 to raise themselves is further enhanced. Such behavior of the side flaps 25 becomes further significant as the front edge 21 and the rear edge 22 elastically contract. The side flaps 25 having such behavior come in close contact with the wearer's thighs from below with respect to the diaper 1 and thereby effectively prevent bodily fluids from leaking out along the wearer's thighs.

To implement the present invention, a liquid-pervious sheet material such as a nonwoven fabric or a perforated plastic film may be used as the inner sheet 2. A liquid-impervious sheet material such as a plastic film or a composite sheet consisting of a plastic film and a nonwoven fabric may be used as the outer sheet 3. Fluff pulp fibers or a mixture of fluff pulp fibers and super-absorbent polymer particles wrapped with an appropriate sheet material such as a tissue paper may be used as the liquid-absorbent core 4. A hydrophobic sheet material such as a nonwoven fabric or a plastic film, more preferably, a hydrophobic and liquid-impervious sheet material may be used as the innermost sheet member 20. While the innermost sheet member 20 are preferably provided along the front edge 21 and the rear edge 22 with the elastic members 21b, 22b attached under tension thereto so that the innermost sheet member 20 may be elastically contracted, it is possible to eliminate use of the elastic members 21b, 22b by using an elastic sheet material adapted to be elastically and contracted in the transverse direction B as the innermost sheet member 20. It should be appreciated that the present invention includes an embodiment according to which both the front edge 21 and the rear edge 22 are not elastically contractible in the transverse direction B.

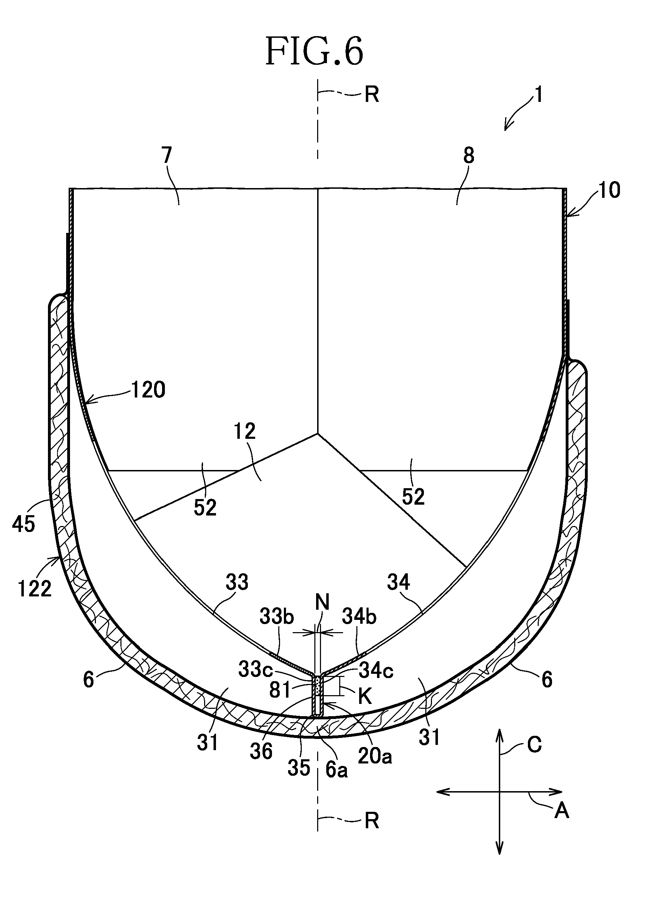

Fig. 5 is a partially cutaway perspective view of one preferred embodiment of the diaper 1 according to the present invention. Regions and members of this embodiment similar to those in the diaper 1 of Fig. 1 are designated with similar reference numerals. Specifically, the diaper 1 of Fig. 5 also has the crotch region 6, the front waist region 7 and the rear waist region 8 wherein the side edges 7a, 7a and 8a, 8a of the front and rear waist regions 7, 8 are put flat and sealed together at the joints 9 arranged intermittently in the vertical direction to form the waist-opening 11 and a pair of the leg-openings 12. The diaper 1 according to this embodiment also includes the innermost sheet member 120 facing the wearer' skin (not shown) and a bodily fluid absorbent member 122 attached to the outer surface 120b of the innermost sheet member 120 wherein these innermost sheet member 120 and the bodily fluid absorbent member 122 are integrated with each other to form the pant-shaped chassis 10. The innermost sheet member 120 is preferably hydrophobic, more preferably hydrophobic and liquid-impervious. Elastic sheet pieces 52 are bonded under tension to the inner surface of the innermost sheet member 120 in the front waist region 7 and the rear waist region 8, respectively, so that the innermost sheet member 120 may be elastically contracted in the circumferential direction. The innermost sheet member 120 is formed in the crotch region 6 with the front opening 33 and the rear opening 34. The front opening 33 has the peripheral edge 33b and is positioned in front of the transverse center line Q-Q so that the wearer's external genital can be exposed in this front opening 33. The rear opening 34 has the peripheral edge 34b and is positioned behind the transverse center line Q-Q so that the wearer's anus can be exposed in this rear opening 34. Referring to Fig. 5, the deformation restricting sheet member 81 interposed between the lower segment 34b of the peripheral edge 34b and the lower segments 34c of the peripheral edge 34b both of the innermost sheet member 120 to join these two segments 33c, 34c is shown (See Fig. 6 also) The imaginary lines J indicate the potential behavior of the bodily fluid-absorbent structure 122 in the crotch region 6 moving inward in opposite directions of the arrows E and F and thereby being crooked so as to be pressed in a direction of the arrow G against the diaper wearer's skin. The deformation restricting sheet member 81 is effective to restrict such potential behavior.

The bodily fluid absorbent member 122 has the bodily fluid absorbent structure 45 comprising the liquid-pervious inner sheet 2, the liquid-impervious outer sheet 3 and the bodily fluid absorbent core 4 sandwiched the inner and outer sheets 2, 3. These inner and outer sheets 2, 3 extend outward beyond the periphery of the core 4 and put flat and bonded together outside the periphery of the core 4 wherein at least one of these inner and outer sheets 2, 3 is bonded to the innermost sheet member 120 so that the front opening 33 and the rear opening 34 may be covered with said one of the inner and outer sheets 2, 3 from outside of the innermost sheet member 120. Along the side edges 13 of the crotch region 6, the inner sheet 2, the outer sheet 3 and the innermost sheet member 120 are placed one on another and bonded together. Also along the side edges 13, the leg elastic members 14b sandwiched between the inner sheet 2 and the outer sheet 3 is bonded under tension to at least one of these two sheets 2, 3. Between the side edges 13, the inner sheet 2 and the innermost sheet member 120 are not bonded to each other so that the innermost sheet member 120 may be spaced from the inner sheet 2 in the thickness direction of the core 4 to form the body waste retaining space 31 into which urine flows through the front opening 3 and feces moves through the rear opening 34.

Fig. 6 is a sectional view taken along the line VI-VI in Fig. 1 wherein the line VI-VI corresponds to the center line P-P extending in the front-back direction. The innermost sheet member 120 is indirectly bonded to itself along the lower segment 33c of the peripheral edge 33b defining the front opening 33 and the lower segment 34c of the peripheral edge 34b defining the rear opening 34 via the deformation restricting sheet member 81 having the thickness N and the height K. The portion 36 of the innermost sheet member 120 defined in the front-back direction A between the lower segments 33c, 34c sags down toward the lowermost portion 6a of the crotch region 6. The portion 36 of the innermost sheet member 120 extending between the lower segments 33c, 34c in the front-back direction A extends downward toward the lower most portion 6a of the crotch region 6 and the lower end 35 of the innermost sheet member 120 is joined to the inner sheet 2.

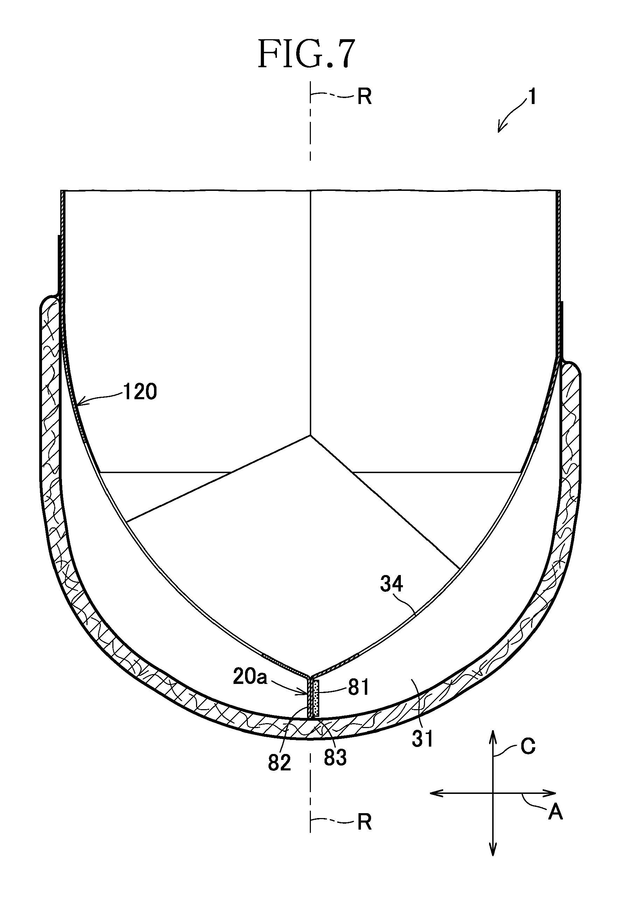

Fig. 7 is a view similar to Fig. 6, showing another preferred embodiment of the present invention. In the diaper 1 shown by Fig. 7, the innermost sheet member 120 is directly bonded to itself to form the separator 20a. The deformation restricting sheet member 81 is attached to the rear surface 83 of the separator 20a. With this alternative arrangement, there is possibility that loose passage flowing into the body waste retaining space comes in direct contact with the deformation restricting sheet member 81. Considering such possibility, it will be advantageous to form the deformation restricting sheet member 81 by a sponge having interconnected cells or a sheet material having many voids such as a nonwoven fabric of crimped conjugate fibers since loose passage can be trapped by these voids. By trapping loose passage of high fluidity in the voids, loose passage would be prevented from flowing out of the rear opening and soiling the wearer's skin. It should be appreciated here that the deformation restricting sheet member 81 may be attached not to the rear surface 83 but to the front surface 82 of the separator 20a without departing from the scope of the present invention.

## Claims

1. A disposable pants-type diaper having a front-back direction, a transverse direction and a vertical direction being orthogonal one to another and comprising a crotch region, a front waist region extending forward from said crotch region and a rear waist region extending rearward from said crotch region wherein said crotch region includes a bodily fluid absorbent structure comprising a liquid-pervious inner sheet, a liquid-impervious outer sheet and a bodily fluid absorbent core sandwiched between said inner and outer sheets and curved with inner sheet inside and said crotch region is additionally formed with a separator adapted to separate urine and feces from each other, said disposable pants-type diaper being **characterized in that:**
the innermost sheet member bonded to said inner sheet to be interposed between skin of wearer of said diaper and said inner sheet is adapted to be partially spaced upward from said inner sheet in said vertical direction and thereby to form a body waste retaining space having a front opening allowing urine to flow into said body waste retaining space and a rear opening allowing feces to move into said body waste retaining space;
a portion of said innermost sheet member extending in said front-back direction between said front opening and said rear opening is folded to be opposed to each other in said front-back direction and directly or indirectly bonded to itself along a center line extending in said transverse direction and bisecting a dimension of said diaper in said front-back direction so that said portion droops down toward said crotch region and a lower end thereof is joined to said inner sheet to form said separator; and
said separator is provided along said center line with a sheet member having a thickness in said front-back direction in a range of 1 to 10mm, a width in said transverse direction in a range of 10 to 70mm and a height in said vertical direction in a range of 30 to 100% of a dimension of said separator in said vertical direction and functioning to restrict a degree of deformation potentially occurring in said separator formed of said innermost sheet material.

2. The diaper defined by Claim 1 wherein said sheet member has a level of stiffness in a range of 40 to 130N based on Procedure D of Stiffness Measuring Method specified by Section 6 of JIS K 6400-2.

3. The diaper defined by Claim 1 or 2 wherein said sheet member is attached to said separator on a front surface of said separator, a rear surface of said separator or between said opposed and joined portions of said innermost sheet member.
